(19) Europäisches Patentamt
European Patent Office
Office européen des brevets

(11) Publication number: **0 362 578 A1**

(12) # EUROPEAN PATENT APPLICATION

(21) Application number: 89116857.7

(22) Date of filing: 12.09.89

(51) Int. Cl.5: **A61K 37/02 , //(A61K37/02, 31:47)**

(30) Priority: **14.09.88 US 244171**

(43) Date of publication of application:
**11.04.90 Bulletin 90/15**

(84) Designated Contracting States:
**AT BE CH DE ES FR GB IT LI LU NL SE**


(71) Applicant: **E.I. DU PONT DE NEMOURS AND COMPANY**
**1007 Market Street**
**Wilmington Delaware 19898(US)**

(72) Inventor: **Loveless, Scott Edward**
**119 Country Club Drive**
**Newark, Delaware 19711(US)**

(74) Representative: **von Kreisler, Alek, Dipl.-Chem. et al**
**Patentanwälte Von Kreisler-Selting-Werner**
**Deichmannhaus am Hauptbahnhof**
**D-5000 Köln 1(DE)**


(54) **Treatment of cancer in mammals by concurrent administration of 4-quinoline carboxylic acid derivatives and interleukin-2.**


(57) Interleukin-2 (IL-2) in combination with 4-quinoline carboxylic acids and derivatives thereof, such as 2-(2′-fluoro-1,1′-biphenyl-4-yl)-6-fluoro-3-methyl-4-quinoline carboxylic acid, are useful for the treatment of solid tumors in mammals. The tumor-inhibiting effect of the combination of IL-2 and quinoline carboxylic acids exceeds the effect of either agent alone.


FIGURE 1

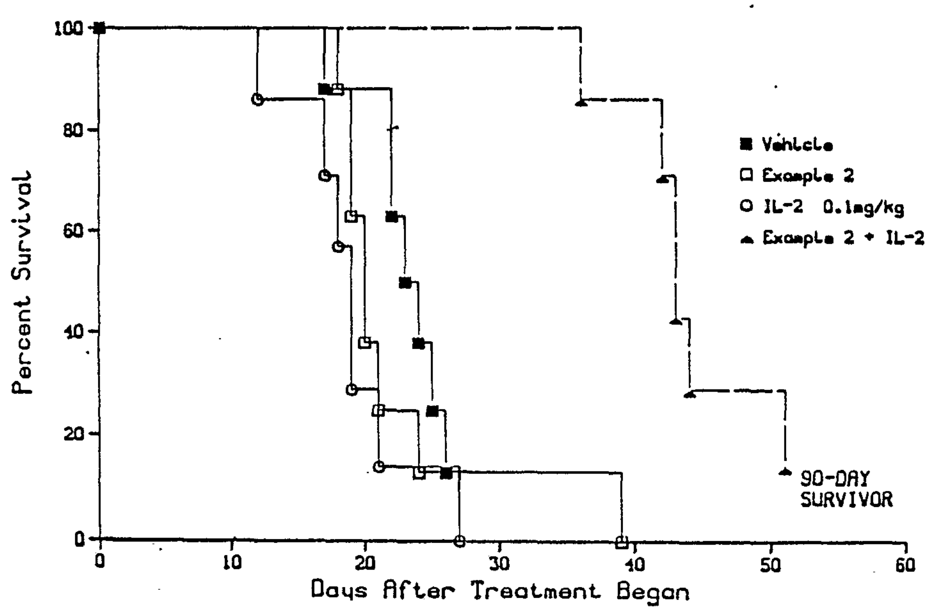


EP 0 362 578 A1

# Treatment of Cancer in Mammals by Concurrent Administration of 4-Quinoline Carboxylic Acid Derivatives and Interleukin-2

## BACKGROUND OF THE INVENTION

This invention relates to methods of treating solid tumors in mammals and more particularly to methods of treating such tumors with a combination of interleukin-2 (IL-2) and 4-quinoline carboxylic acids, and derivatives thereof.

IL-2 is a protein secreted by lymphocytes and belongs to the class of immune modulating substances called lymphokines. IL-2 was first described as T-cell growth factor (TCGF), a lymphokine capable of promoting the proliferation of T lymphocytes (Morgan et al. (1976) Science 193, 1007-1008; Ruscetti et al. (1977) J. Immunol. 119, 131-138). IL-2 has been shown to modulate many other immunological effects on lymphoid cells, including cytotoxic T-cells, natural killer cells, activated B-cells, and lymphokine activated killer (LAK) cells (Robb (1984) Immunol, Today 5, 203 and references therein). IL-2 is used to generate LAK cells that kill fresh tumor cells but not normal cells (Grimm et al. (1982) J. Exp. Med. 155, 1823-1841; Mazumder et al. (1984) J. Exp. Med. 159, 495-507). Recently, treatment of cancer patients by administration of IL-2 and autologous LAK cells has demonstrated the potential use of IL-2 as an immunotherapeutic agent (Rosenberg et al. (1985) N. Eng. J. Med. 313, 1485-1492; U.S. Patent 4,690,915).

Molecular cloning of the human IL-2 cDNA (Taniguchi et al. (1983) Nature 302, 305-310; U.S. Patent 4,738,927; Deves et al. (1983) Nucleic Acids Res. 11, 4307-4323) has allowed determination of the primary sequence structure of IL-2 and the production of large amounts of biologically functional recombinant IL-2 using heterologous expression systems, including E. coli systems (Ju et al. (1987) J. Biol. Chem. 262, 5723-5731 and references therein).

Extremely high doses of IL-2 can bring about antitumor effects in the mouse (Rosenberg et al. (1985) J. Exp. Med. 161, 1169-1188). Thus, in the mouse, administration of high doses of recombinant IL-2 alone have antitumor effects. However, it is not possible to achieve these high doses of IL-2 in humans because of the toxicity associated with IL-2 administration. Indeed, in previous human studies in which 39 humans received low doses of IL-2 alone (16 received the natural human IL-2 and 23 received recombinant IL-2), no antitumor effects were seen (Rosenberg (1984) J. Biol. Resp. Med. 3, 501-511; Mazumder et al. (1984) Cancer 53, 896-905; Lotze et al. (1985) J. Immunol, 134, 157-166). Clearly, the therapeutic efficacy of IL-2 for the treatment of human cancer is limited by the severe toxicity of IL-2 when administered at high doses, i.e., doses greater than about $4\times10^6$ international (BRMP) units per kg body weight.

IL-2 toxicity in humans includes excessive fluid retention, probably resulting from a generalized increase in capillary permeability, fever, malaise, generalized erythematous rash, gastrointestinal bleeding, renal dysfunction, eosinophilia, and hyperbilirubinema. IL-2 administration is also associated with hematopoietic suppression, necessitating blood transfusion to correct anemia.

U.S. Patent 4,680,299, granted July 14, 1987 to Hesson, describes phenylquinoline carboxylic acids and their derivatives as agents which inhibit tumor growth.

It has now been found that the tumor-inhibiting effect of IL-2 in mammals is enhanced when IL-2 is administered in conjunction with the compounds described in U.S. 4,680,299. In particular, the concurrent use of quinoline carboxylic acids and IL-2 for the treatment of cancer in a mammal greatly increases the effective therapeutic efficacy of IL-2, allowing the use of lower doses of IL-2.

## SUMMARY OF THE INVENTION

According to the present invention there is provided a method of treating a solid tumor in a mammal comprising administering concurrently to the mammal a tumor-inhibiting amount of IL-2 and a compound having the formula:

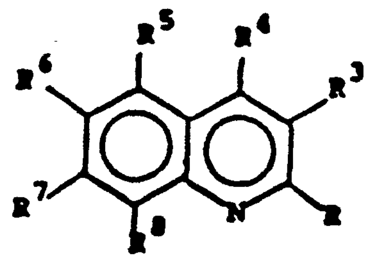

(I)

wherein

R is 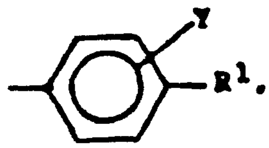 , 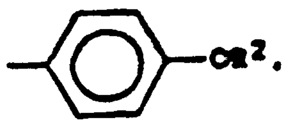 , 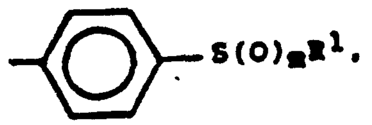 , or

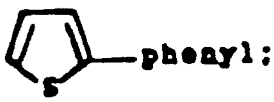

R' is $CH_3CH_2(CH_3)CH$, alkyl of 5-12 carbon atoms, cyclohexyl,

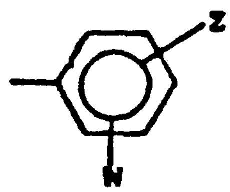 or 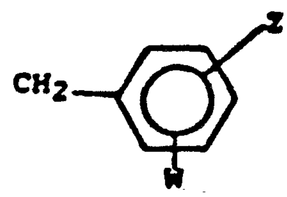

when R is

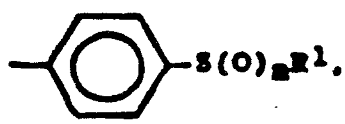

$R^1$ can be in addition alkyl of 3-4 carbon atoms;
$R^2$ is

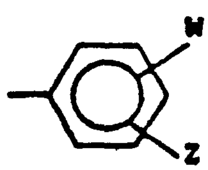 or 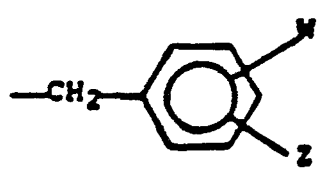

$R^3$ is H, alkoxy of 1-3 carbon atoms, or alkyl of 1-2 carbon atoms;
$R^4$ is $CO_2H$ or $CO_2R^{11}$;
$R^5$, $R^6$, $R^7$ and $R^8$ are independently H, F, Cl, Br, I, $CH_3$, $CF_3$, $SCH_3$ or $CH_2CH_3$, at least two of $R^5$, $R^6$, $R^7$ and $R^8$ being H;
$R^9$ and $R^{9A}$ are independently H or alkyl of 1 to 3 carbon atoms;
$R^{11}$ is $(CH_2)_{2-4}NR^9R^{9A}$;
W, Y and Z are independently H, F, Cl, Br, alkyl of 1-5 carbon atoms, $NO_2$, OH, $CF_3$ or $OCH_3$;
m is 0 or 1; or
a pharmaceutically suitable salt thereof;
with the following provisos:

(1) $R^5$, $R^6$ and $R^7$ cannot all be H;
(2) when $R^4$ is $CO_2CH_2CH_2N(CH_3)_2$, $R^6$ is $CH_2CH_3$, or $R^7$ is Cl, $R^1$ cannot be cyclohexyl;
(3) when $R^1$ is cyclohexyl and $R^3$ is H, $R^6$ must be Cl or F, but $R^6$ and $R^8$ cannot both be Cl; and
(4) when $R^6$ is $CH_3$, then $R^7$ cannot be Cl.

The administration of IL-2 in conjunction with quinoline carboxylic acids, and derivatives thereof, as described herein, represents a new approach to the treatment of cancer with potential applicability to a wide variety of tumors.

## PREFERRED EMBODIMENTS

A preferred IL-2 is rIL-2 having a specific activity in the range of $3x10^6$ to $2x10^7$ international (BRMP) units/mg.

Preferred compounds useful in the method have the formula:

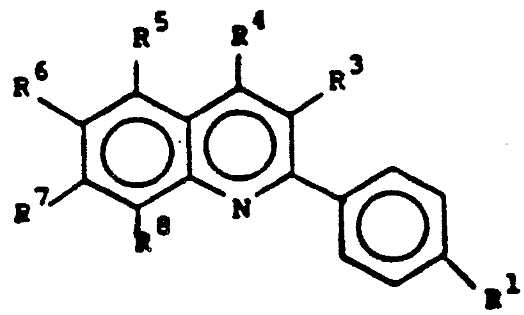

(II)

wherein
$R^1$ is cyclohexyl; phenyl; phenyl substituted with one halogen; alkyl of 1-5 carbon atoms of $CF_3$; phenoxy; or phenoxy substituted with one halogen or alkyl of 1-5 carbon atoms;
$R^3$ is H or alkyl of 1-2 carbon atoms;
$R^4$ is $CO_2H$, a sodium or potassium salt thereof; or $CO^2R^{11}$;
$R^5$ and $R^6$ are independently H, halogen, $CH_3$ or $CF_3$;
$R^7$ and $R^8$ are independently H or halogen;
$R^{11}$ is $(CH_2)_{2-4}NR^9R^{9A}$; and
$R^9$ and $R^{9A}$ are independently alkyl of 1 to 3 carbon atoms,
or a pharmaceutically suitable salt thereof;
provided that $R^5$, $R^6$ and $R^7$ cannot all be H and that when $R^1$ is cyclohexyl and $R^3$ is H, $R^6$ must be Cl or F, but $R^6$ and $R^8$ cannot both be Cl, and when $R^6$ is $CH_3$, then $R^7$ cannot be Cl.

More preferred compounds useful in this invention have the formula:

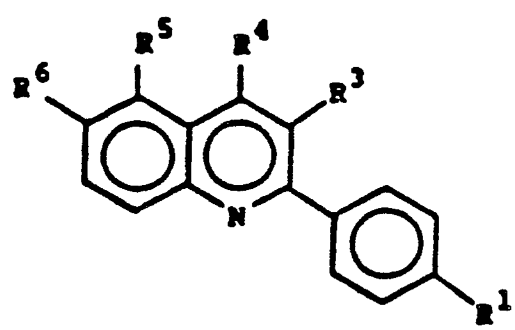

(III)

wherein
$R^1$ is cyclohexyl,

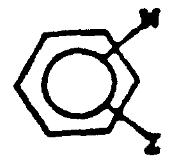 or 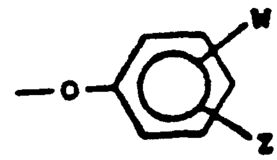

$R^3$ is H or alkyl of 1-2 carbon atoms;

$R^4$ is $CO_2H$, a sodium or potassium salt thereof, or $CO_2R^{11}$;

$R^5$ and $R^6$ are independently H, halogen or $CF_3$ provided that both $R^5$ and $R^6$ are not hydrogen;

$R^{11}$ is $(CH_2)_{2-4}NR^9R^{9A}$; and

$R^9$ and $R^{9A}$ are independently alkyl of 1 to 3 carbon atoms, and

W and Z are independently H, halogen, alkyl of 1-5 carbon atoms or $CF_3$;

provided that when $R^1$ is phenyl or phenoxy, and $R^5$ is H, then $R^6$ cannot be Br; and that when $R^1$ is cyclohexyl and $R^3$ is H, $R^6$ must be Cl or F.

Specifically preferred compounds useful in this invention are:

(1) 2-(1,1′-biphenyl-4-yl)-5-chloro-3-methyl-4-quinoline carboxylic acid, sodium or potassium salt

(2) 2-(1,1′-biphenyl-4-yl)-6-fluoro-3-methyl-4-quinoline carboxylic acid, sodium or potassium salt

(3) 6-fluoro-3-methyl-2-(4-phenoxyphenyl)-4-quinoline carboxylic acid, sodium or potassium salt

(4) 2-(4′-bromo-1,1′-biphenyl-4-yl)-6-fluoro-3-methyl-4-quinoline carboxylic acid, sodium or potassium salt

(5) 2-(2′-fluoro-1,1′-biphenyl-4-yl)-6-fluoro-3-methyl-4-quinoline carboxylic acid, sodium or potassium salt.

## BRIEF DESCRIPTION OF THE FIGURES

Figure 1 is a graph showing the effect of the compound of Example 2 and rIL-2 on the survival of mice bearing intradermally implanted B16 melanoma.

Figure 2 is a graph showing the effect of the compound of Example 2 and rIL-2 on the survival of mice bearing intraperitoneally implanted B16 melanoma.

Figure 3 is a graph showing the effect of the compound of Example 2 and rIL-2 on the growth of B16F10 melanoma lung metastases.

## DETAILED DESCRIPTION OF THE INVENTION

The compounds useful in this invention are described in and prepared by methods set forth in U.S. Patent 4,680,299, the disclosure, synthesis, and synthesis examples of which are hereby incorporated by reference.

The IL-2 useful in this invention can be either in its natural form (U.S. Patent 4,401,756, issued to Gillis on August 30, 1983; U.S. Patent 4,490,289, issued to Stern on December 25, 1985) or in a recombinant form (U.S. Patent 4,738,927 issued to Taniguchi et al. on April 17, 1988; coassigned U.S. Serial No. 825,133, filed January 1, 1986 in the name of Tamblyn).

The invention can be further understood by the following examples in which parts and percentages are by weight unless otherwise indicated; all temperatures are in degrees centigrade.

### Example 1

#### Part A: 2-(1,1′-Biphenyl-4-yl)-5-chloro-3-methyl-quinoline-4-carboxylic acid

A mixture of 4-chloroisatin (7.28 g, .04 mol), [J. Am. Chem. Soc., 1251 (1956)], 4-phenylpropiophenone (8.8 g, .04 mol), diethylamine (4 ml, .04 mol) and ethanol (200 ml) was stirred for a period of 18 hours at room temperature. The precipitated solids were collected by filtration, washed with ice-cold ethanol and air

dried to yield the adduct (9.1 g, 58%), m.p. 209-214° dec.

Part B:

The above described adduct (9.1 g) was added to a mixture of tetrahydrofuran (200 ml), and concentrated HCl (200 ml) and heated at reflux for 24 hr. The reaction mixture was cooled, water (300 ml) was added and most of the tetrahydrofuran removed by evaporation in vacuo. The aqueous residue was cooled and the sticky solids collected by filtration. Trituration in 150 ml of boiling methanol yielded (4.8 g, 55%) m.p. 295-297° dec.
$C_{23}H_{16}ClNO_2$ HRMS: 373,0869 Calcd, measured m/e 373.0814.
$^{1}H$ NMR (DMSO-$d_6$):$\delta$ 8.5(m,1H), 7.7-7.85(m,7H), 7.35-7.55(m,4H), 2.45(s,3H).

Part C: Sodium 2-(1,1′-Biphenyl-4-yl)-5-chloro-3-methyl-quinoline-4-carboxylate

To a suspension of the above acid (3.7 g, .01 mol) in ethanol 100 ml, sodium hydroxide (1N, 10 ml, .01 mol) was added, and gently warmed. The clear solution was then filtered and evaporated to dryness to yield (4.0 g) m.p. 320-330° dec.

## Example 2

Part A: 2-(2-Fluoro-1,1′-biphenyl-4-yl)-6-fluoro-3-methyl-4-quinoline carboxylic acid

5-Fluoroisatin (72.6 g, 0.44 mole) and 4-(2-fluorophenyl)propiophenone (100 g, 0.44 mole) were suspended in 720 ml of ethanol and stirred mechanically as a solution of KOH (147.8 g, 2.64 mole) in 300 ml of water was added dropwise over 15 minutes. The reaction mixture was heated at reflux for 12 hours, cooled and the ethanol evaporated under reduced pressure. The resulting solid was dissolved in water and washed with ethyl ether. The aqueous layer was cooled to 5° and acidified with glacial acetic acid. The resulting precipitate was filtered, washed 2 times with 300 ml of ethyl ether and dried. Recrystallization from dimethylformamide and water gave 84 g of a white 2-(2′-Fluoro-1,1′-biphenyl-4-yl)-6-fluoro-3-methyl-4-quinoline carboxylic acid, m.p. 315°-317°.

Part B: Sodium 2-(2′-Fluoro-1,1′-biphenyl-4-yl)6-fluoro-3-methylquinoline-4-carboxylate

The compound of Part A (37.5 g, 0.10 mole) was suspended in 1,000 ml of ethanol and treated with 1N NaOH (100 ml, 0.10 mole). The mixture was warmed and stirred until clear; the ethanol and water were evaporated at reduced pressure to give 39.6 g of the white solid sodium 2-(2′-fluoro-1,1′-biphenyl-4-yl)6-fluoro-3-methylquinoline-4-carboxylate, m.p. >360°.

Following the procedures of Examples 1 and 2 or the synthesis procedures described in U.S. 4,680,299, the compounds set forth in Table 1 were prepared.

## Table 1

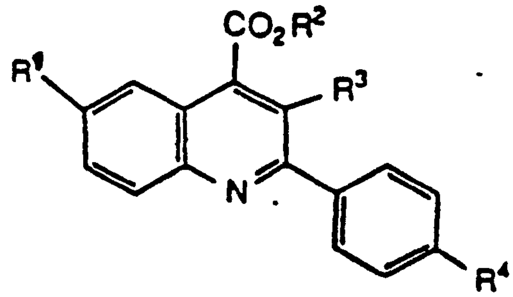

| Ex. No. | $R^1$ | $R^2$ | $R^3$ | R4 | m.p.(°) |
|---|---|---|---|---|---|
| 3 | F | Na | $CH_3$ | 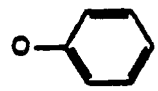 | >350 |
| 4 | F | Na | $CH_3$ | 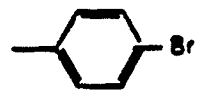 | >350 |
| 5 | $CH_3$ | Na | $CH_3$ | 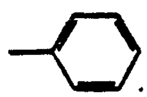 | >350 |
| 6 | F | Na | $CH_3$ | $-S-CH(CH_3)_2$ | 339-343 |
| 7 | Cl | Na | $CH_3$ | 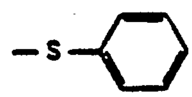 | 319-324 |
| 8 | Cl | K | $CH_3$ | 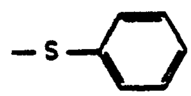 | 310-325 |
| 9 | F | Na | H | 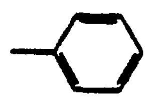 | >360 |
| 10 | F | Na | $CH_3$ | 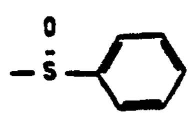 | 251-260 |
| 11 | F | Na | $OCH_3$ | 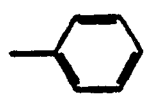 | 345-349 |
| 12 | Cl | Na | $CH_3$ | 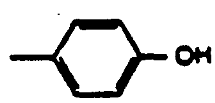 | >360 |

### Interleukin-2

The recombinant E. coli-expressed IL-2 used in the present study was prepared by E. I. du Pont de Nemours & Company. E. coli-expressed mature human IL-2 was expressed using the plasmid vector

pTrpE-IL2, such a product of pTrpE-IL2 being designated rIL-2. The construction of plasmid pTrpE-IL2 and the expression in E. coli and purification of recombinant mature human IL-2 are described in coassigned U.S. Ser. No. 07/128,931, filed December 4, 1987 in the name of Simon et al. and U.S. Ser. No. 825,133, filed January 1, 1986 in the name of Tamblyn, which are hereby incorporated by reference.

rIL-2 differs from mature human cell-expressed IL-2 (Taniguchi et al. (1983) Nature 302, 305-310; Deves et al. (1983), Nucleic Acid Res. 11, 4307-4323) by the addition of an N-terminal Met residue that is present in approximately 50% of the rIL-2 molecules.

The rIL-2 was purified to homogeneity and migrated as a single band on SDS polyacrylamide gel electrophoresis. rIL-2 was obtained as a lyophilized powder and was reconstituted with 1.2 ml of sterile water per vial. Each vial contained approximately 0.3 mg of rIL-2 (specific activity = $3x10^6$ to $2x10^7$ international (BRMP) units/mg. Less than 0.04 nanograms of endotoxin were present per vial as measured by the limulusamebocyte assay. Each vial also contained 5% mannitol and approximately 130 µg of sodium dodecyl sulfate per mg of IL-2.

Recombinant or nonrecombinant IL-2, obtained from any suitable source, can also be used.

## Utility

Results of the biological tests described below establish that the compounds administered according to this invention have the property of inhibiting the growth of transplanted solid mouse tumors in mice.

The efficacy of the compounds of this invention against the transplanted mouse tumors was evaluated in test systems currently in use at the National Cancer Institute for the detection and assessment of anticancer activity. Most clinically effective drugs exhibit activity in these tests and the tests have a good record of predicting clinical efficacy (Goldin et al. (1981) Europ. J. Cancer 17, 129-142; Venditti (1981) Seminars in Oncology 8, 4; Goldin and Venditti, in Recent Results in Cancer Research 70, S. K. Carter and Y. Sakurai, Eds., Springer-Verlag, Berlin/Heidelberg, 1980).

## B16 Melanotic Melanoma Test

The animals used were $B_6C_3F_1$ mice, all females, weighing a minimum of 18 g and all within a 4 g weight range at the start of the test. The test group comprised 8 or 10 mice. The B16 melanoma was implanted in each of the test mice by either intradermal injection of 0.05 ml of a 40% Brei, or by intraperitoneal injection of 0.5 ml of a 10% Brei prepared by homogenizing fresh melanoma tumors in cold physiological saline. The test compounds were suspended in 0.3 M glucose. In the intradermal study, 5 mg/kg of the compound of Example 2 was administered once daily for nine consecutive days starting 12 days after tumor implantation. The IL-2, in this experiment, was administered twice daily at a dose of 0.1 mg/kg on days 1-5 and twice daily on days 8-12 starting 12 days after tumor implantation. In the intraperitoneal study, the compound of Example 2 was administered once daily at a dose of 5 mg/kg for 9 consecutive days starting 1 day after tumor implantation. The IL-2 in the intraperitoneal experiment was administered twice daily at a dose of 0.1 mg/kg for 9 consecutive days starting 1 day after tumor implantation. The control mice received injectins of 0.3 M glucose only. The mice were weighed and survivors were recorded on a regular basis for 90 days. The effect of the treatment of mice bearing intradermally or intraperitoneally implanted B16 melanoma with rIL-2 alone, the compound of Example 2 alone, or the combination of rIL-2 and the compound of Example 2 is shown in Figures 1 and 2.

As can be seen, the survival rate of mice receiving both compounds exceeds the survival rate for mice receiving each compound alone.

## Antimetastatic Activity of Compounds Using the Experimental B16F10

## Melanoma Metastasis Assay in Mice

Death from cancer is primarily due to uncontrolled growth of tumor cells to secondary organs, i.e., metastases. Since metastases develop after gaining entry into the circulation, invading the wall of the arrested vessel, infiltration into adjacent tissue, and subsequent growth and vascularization, an experimental metastasis model is used. In this model, B16F10 melanoma cells are injected intravenously in mice, the mice are treated with drugs, lungs are removed, and melanotic nodules enumerated (Fidler, in Methods in Cancer Research, Vol. XV, pp. 399-439, 1978).

C57BL6 mice were inoculated with tumor cells by intravenous injection with 0.2 ml containing 1.25 x $10^5$ ml in Hanks balanced salt solution.

Following tumor injection (day 0), mice were injected with vehicle alone (0.3 M glucose) or treated by intraperitoneal injection of the compound of Example 2 alone (dose = 25 mg kg), rIL-2 alone (dose = 1 mg kg), or both the compound and rIL-2, once daily for nine consecutive days starting on day 1. About two weeks after tumor cell inoculation, on the day before sacrifice, each mouse received an intravenous injection of 0.2 ml containing $10^{-5}$M fluorodeoxyuridine and 2 $\mu$Ci [$^{125}$I]iododeoxyuridine. Twenty-four hours later, the mice were sacrificed and the lungs were removed and counted in a gamma counter. Manual counting of metastases using a dissecting microscope can also be done if needed.

The effect of the treatment of mice inoculated with B16F10 melanoma with rIL-2 alone, the compound of Example 2 alone, or the combination of rIL-2 and the compound of Example 2, is shown in Figure 3. As shown in Figure 3, the effect of the compound of Example 2 in conjunction with rIL-2 exceeds the effect of either agent alone. In the experiment shown in Figure 3, the compound of Example 2 in conjunction with rIl-2 resulted in a >95% inhibition of the tumor growth.

## Dosage Forms

The antitumor compounds (active ingredients) useful in this invention can be administered to treat solid tumors by any means that produces contact of the active ingredient with the agent's site of action in the body of a mammal. They can be administered by any conventional means available for use in conjunction with pharmaceuticals; either as individual therapeutic active ingredients or in a combination of therapeutic active ingredients. They can be administered alone, but are generally administered with a sterile, nontoxic, nonallergenic, physiologically compatible pharmaceutical carrier selected on the basis of the chosen route of administration and standard pharmaceutical practice.

The dosage administered will be a tumor-inhibiting amount of active ingredient and will, of course, vary depending upon known factors such as the pharmacodynamic characteristics of the particular active ingredient, and its mode and route of administration; age, health, and weight of the recipient; nature and extent of symptoms; kind of concurrent treatment, frequency of treatment, and the effect desired.

Administration of the IL-2 to humans or animals may be oral or intraperitoneal or intramuscular or subcutaneous as deemed appropriate by the physician. The amount of IL-2 administered will usually range between about $3.3x10^3$ to $3.3x10^6$ international (BRMP) units per kg body weight administered multiple times daily. Because murine tumor models suggest that sustained serum levels of IL-2 are necessary to obtain optimal therapeutic effects, increasing doses of IL-2 intravenously may be utilized. For example, patients may receive bolus intravenous injections of IL-2 every 8 hours at doses of either 33,000 units/kg, 99,000 units/kg, or 330,000 units/kg depending on the protocol used in each patient. The specific activity of pure rIL-2 is approximately $10^7$ international (BRMP) units/mg.

Usually a daily dosage of a quinoline carboxylic acid active ingredient can be about 0.1 to 400 milligrams per kilogram of body weight. Ordinarily 1 to 100, and preferably 1 to 50 milligrams per kilogram per day is effective to obtain desired results.

The IL-2 dose can range from $3.3x10^3$ to $3.3x10^6$ international units per kg of body weight, administered multiple times daily.

IL-2 and a quinoline carboxylic acid are preferably administered to the mammal concurrently at about 10% of the maximum tolerated dose of each component which is about $3.3x10^5$ unit kg and 40 mg/kg, respectively.

Dosage forms (compositions) suitable for internal administration contain from about 1 to 500 milligrams of active ingredient per unit. In these pharmaceutical compositions the active ingredient will ordinarily be present in an amount of about 0.5 to 95% by weight based on the total weight of the composition.

The active ingredient can be administered orally in solid dosage forms, such as capsules, tablets, and powders, or in liquid dosage forms, such as elixirs, syrups, and suspensions, it can also be administered parenterally, in sterile liquid dosage forms.

Gelatin capsules contain the active ingredient and powdered carriers, such as lactose, sucrose, mannitol, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration contain preferably a water soluble salt of the active ingredient, suitable stabilizing agents, and if necessary, buffer substances. Antioxidizing agents such as sodium bisulfite, sodium sulfite, or ascorbic acid either alone or combined are suitable stabilizing agents. Also used are citric acid and its salts and sodium EDTA. In addition parenteral solutions can contain preservatives, such as benzalkonium chloride, methyl- or propyl-paraben, and chlorobutanol.

Suitable pharmaceutical carriers are described in Remington's Pharmaceutical Sciences, A. Osol, a standard reference text in this field.

## Capsules

A large number of unit capsules are prepared by filling standard two-piece hard gelatin capsules each with 100 milligrams of powdered active ingredient, 175 milligrams of lactose, 24 milligrams of talc, and 6 milligrams magnesium stearate.

A mixture of active ingredient in soybean oil is prepared and injected by means of a positive displacement pump into gelatin to form soft gelatin capsules containing 100 milligrams of the active ingredient. The capsules are washed and dried.

## Tablets

A large number of tablets are prepared by conventional procedures so that the dosage unit is 100 milligrams of active ingredient, 0.2 milligrams of colloidal silicon dioxide, 5 milligrams of magnesium stearate, 275 milligrams of microcrystalline cellulose, 11 milligrams of cornstarch and 98.8 milligrams of lactose. Appropriate coatings may be applied to increase palatability or delay absorption.

## Injectable

A parenteral composition suitable for administration by injection is prepared by stirring 1.5% by weight of active ingredient in 10% by volume propylene glycol and water. The solution is made isotonic with sodium chloride and sterilized.

## Suspension

An aqueous suspension is prepared for oral administration so that each 5 milliliters contain 100 milligrams of finely divided active ingredient, 200 milligrams of sodium carboxymethyl cellulose, 5 milligrams of sodium benzoate, 1.0 grams of sorbitol solution, U.S.P., and 0.025 milliliters of vanillin.

The same dosage forms can generally be used when the compounds of this invention are administered stepwise in conjunction with another therapeutic agent. When the drugs are administered in physical combination, the dosage form and administration route should be selected for compatibility with both drugs.

## Claims

1. A method of treating a solid tumor in a mammal comprising administering concurrently to the mammal an effective amount of IL-2 and a compound having the formula:

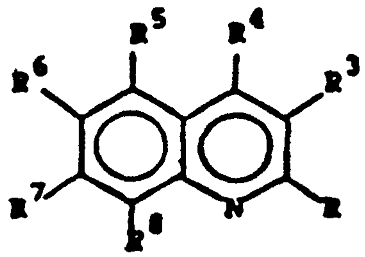

(I)

wherein

R is 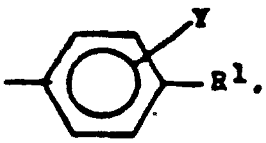, 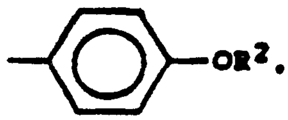, 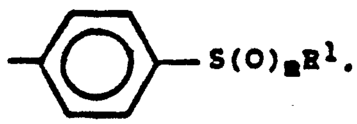, or

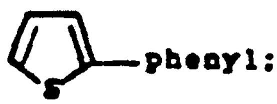

$R^1$ is $CH_3CH_2(CH_3)CH$, alkyl of 5-12 carbon atoms, cyclohexyl,

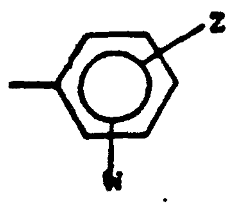 or 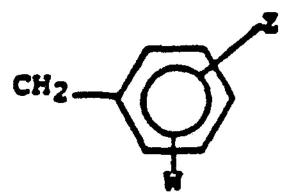

when R is

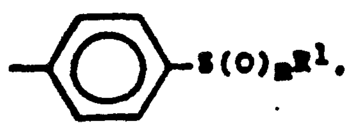

$R^1$ can be in addition alkyl of 3-4 carbon atoms;

$R^2$ is

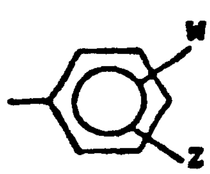 or 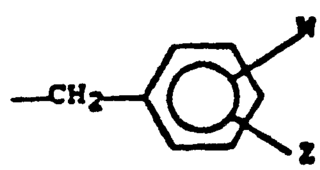

$R^3$ is H, alkoxy of 1-3 carbon atoms, or alkyl of 1-2 carbon atoms;

$R^4$ is $CO_2H$ or $CO_2R^{11}$;

$R^5$, $R^6$, $R^7$ and $R^8$ are independently H, F, Cl, Br, I, $CH_3$, $CF_3$, $SCH_3$ or $CH_2CH_3$, at least two of $R^5$, $R^6$, $R^7$ and $R^8$ being H;

$R^9$ and $R^{9A}$ are independently H or alkyl of 1 to 3 carbon atoms;

$R^{11}$ is $(CH_2)_{2-4}NR^9R^{9A}$;

W, Y and Z are independently H, F, Cl, Br, alkyl of 1-5 carbon atoms, $NO_2$, OH, $CF_3$ or $OCH_3$;
m is 0 or 1; or
a pharmaceutically suitable salt thereof;
with the following provisos:

(1) $R^5$, $R^6$ and $R^7$ cannot all be H;
(2) when $R^4$ is $CO_2CH_2CH_2N(CH_3)_2$, $R^6$ is $CH_2CH_3$, or $R^7$ is Cl, $R^1$ cannot be cyclohexyl;
(3) when $R^1$ is cyclohexyl and $R^3$ is H, $R^6$ must be Cl or F, but $R^6$ and $R^8$ cannot both be Cl; and
(4) when $R^6$ is $CH_3$, then $R^7$ cannot be Cl.

2. The method of Claim 1 wherein the compound has the formula:

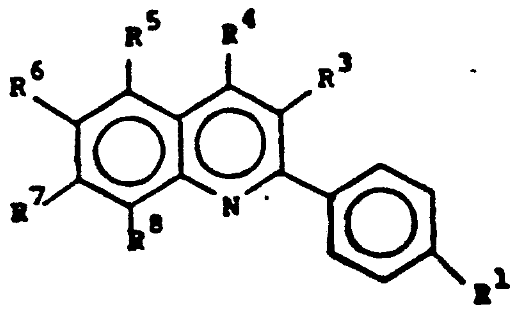

(II)

wherein
$R^1$ is cyclohexyl; phenyl; phenyl substituted with one halogen; alkyl of 1-5 carbon atoms of $CF_3$; phenoxy; or phenoxy substituted with one halogen or alkyl of 1-5 carbon atoms;
$R^3$ is H or alkyl of 1-2 carbon atoms;
$R^4$ is $CO_2H$, a sodium or potassium salt thereof; or $CO^2R^{11}$;
$R^5$ and $R^6$ are independently H, halogen, $CH_3$ or $CF_3$;
$R^7$ and $R^8$ are independently H or halogen;
$R^{11}$ is $(CH_2)_{2-4}NR^9R^{9A}$; and
$R^9$ and $R^{9A}$ are independently alkyl of 1 to 3 carbon atoms,
or a pharmaceutically suitable salt thereof;
provided that $R^5$, $R^6$ and $R^7$ cannot all be H and that when $R^1$ is cyclohexyl and $R^3$ is H, $R^6$ must be Cl or F, but $R^6$ and $R^8$ cannot both be Cl, and when $R^6$ is $CH_3$, then $R^7$ cannot be Cl.

3. The method of Claim 1 wherein the compound has the formula:

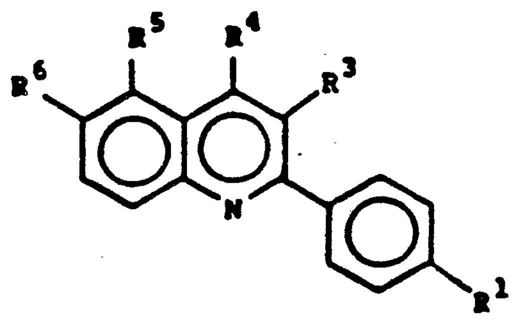

(III)

wherein
$R^1$ is cyclohexyl,

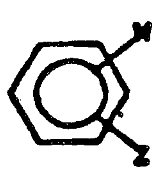

or

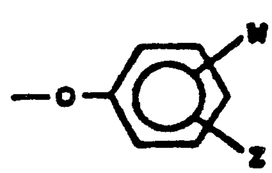

$R^3$ is H or alkyl of 1-2 carbon atoms;

$R^4$ is $CO_2H$, a sodium or potassium salt thereof, or $CO_2R^{11}$;
$R^5$ and $R^6$ are independently H, halogen $CF_3$ provided that both $R^5$ and $R^6$ are not hydrogen;
$R^{11}$ is $(CH_2)_{2-4}NR^9R^{9A}$; and
$R^9$ and $R^{9A}$ are indepentdently alkyl of 1 to 3 carbon atoms, and
W and Z are independently H, halogen, alkyl of 1-5 carbon atoms or $CF_3$;
provided that $R^1$ is phenyl or phenoxy, and $R^5$ is H, then $R^6$ cannot be Br; and that when $R^1$ is cyclohexyl and $R^3$ is H, $R^6$ must be Cl or F.

4. The method of Claim 1 wherein the compound is 2-(1,1′-biphenyl-4-yl)-5-chloro-3-methyl-4-quinoline carboxylic acid, sodium or potassium salt.

5. The method of Claim 1 wherein the compound is 2-(1,1′-biphenyl-4-yl)-6-fluoro-3-methyl-4-quinoline carboxylic acid, sodium or potassium salt.

6. The method of Claim 1 wherein the compound is 6-fluoro-3-methyl-2-(4-phenoxyphenyl)-4-quinoline carboxylic acid, sodium or potassium salt.

7. The method of Claim 1 wherein the compound is 2-(4′-bromo-1,1′-biphenyl-4-yl)-6-fluoro-3-methyl-4-quinoline carboxylic acid, sodium or potassium salt.

8. The method of Claim 1 wherein the compound is 2-(2′-fluoro-1,1′-biphenyl-4-yl)-6-fluoro-3-methyl-4-quinoline carboxylic acid, sodium or potassium salt.

9. The method of Claim 1 wherein the IL-2 is rIL-2.

10. The method of Claim 8 wherein the IL-2 is rIL-2.

11. The method of Claim 1 wherein the IL-2 is administered multiple times daily at a dose in the range of $3.3x10^3$ to $3.3x10^6$ international units per kg and the compound is administered at a dose in the range of 1 to 100 mg/kg.

12. The method of Claim 9 wherein the rIL-2 is administered multiple times daily at a dose in the range of $3.3x10^3$ to $3.3x10^6$ international units per kg and the compound is administered at a dose in the range of 1 to 50 mg/kg.

13. The method of Claim 10 wherein each of rIL-2 and the compound is administered multiple times daily at about 10% of its maximum tolerated dose which is about 0.1 mg/kg of rIL-2 and about 5 mg/kg of the compound.

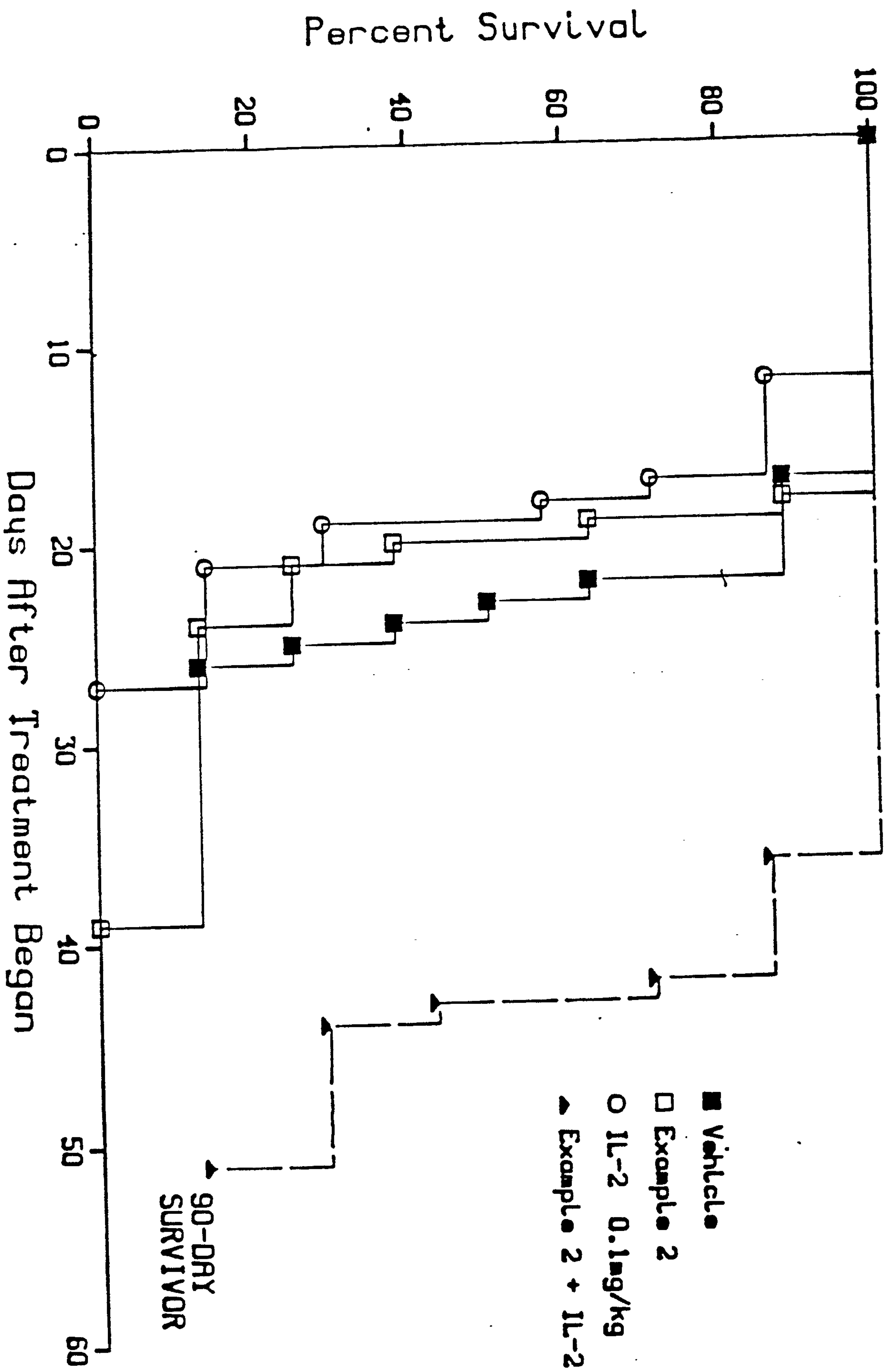
Percent Survival
0
20
40
60
80
100
0
10
20
30
40
50
60
Days After Treatment Began
Vehicle
Example 2
IL-2 0.1mg/kg
Example 2 + IL-2
90-DAY SURVIVOR

FIGURE 1

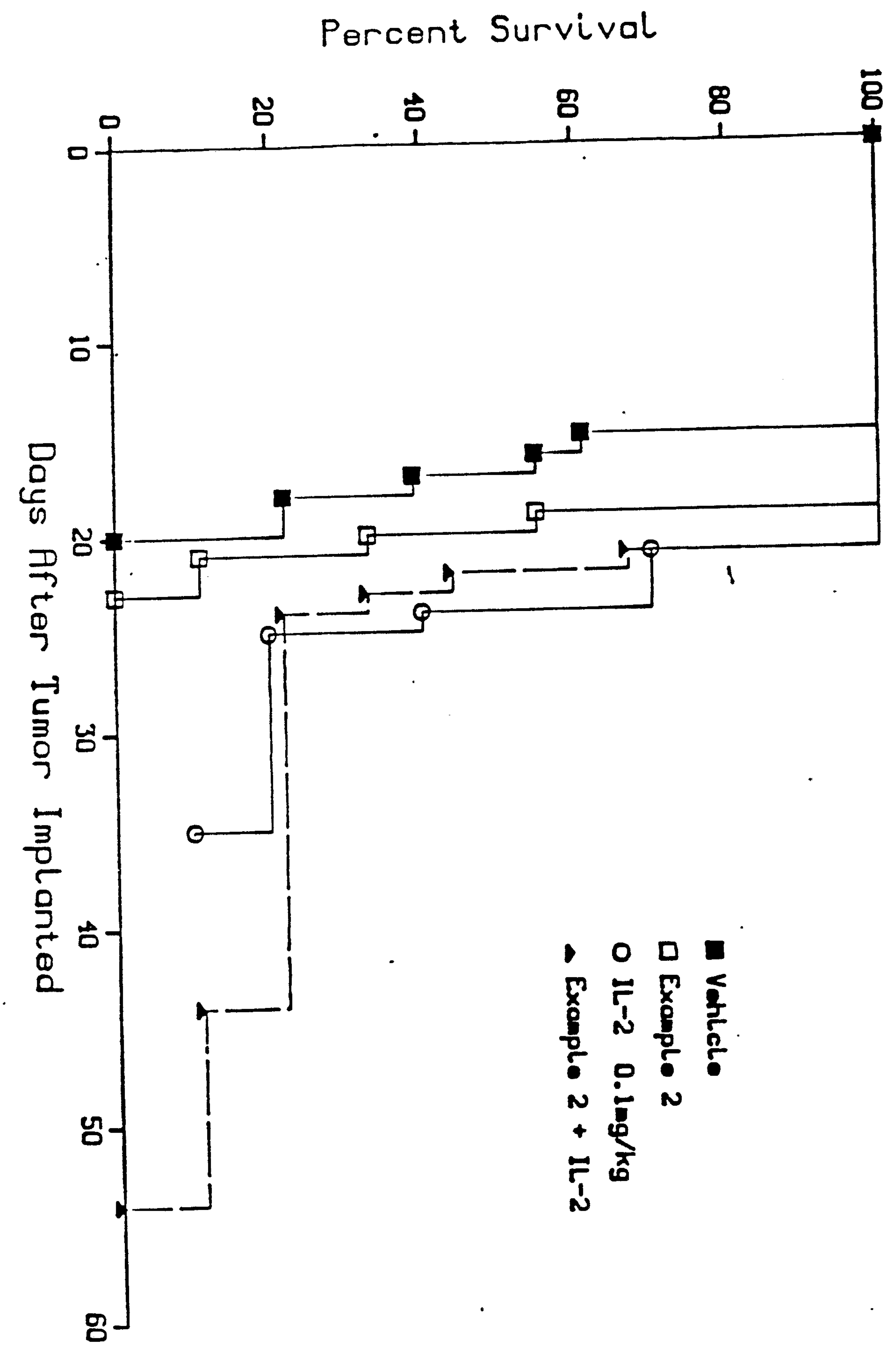
Percent Survival
0
20
40
60
80
100
Days After Tumor Implanted
0
10
20
30
40
50
60
Vehicle
Example 2
IL-2 0.1mg/kg
Example 2 + IL-2

FIGURE 2

# Figure 3

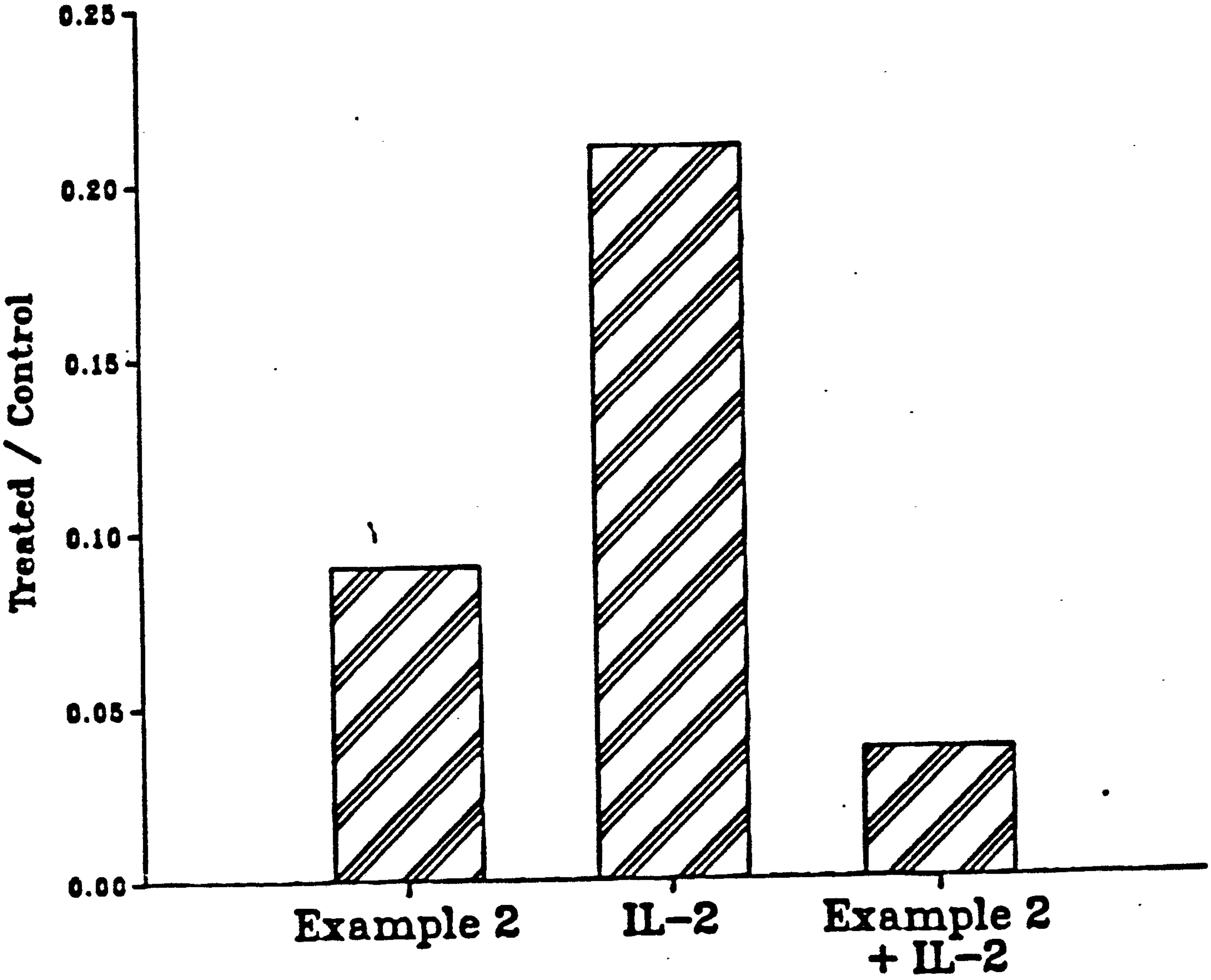
0.25
0.20
0.15
0.10
0.05
0.00
Treated / Control
Example 2
IL-2
Example 2
+ IL-2

European Patent Office

# PARTIAL EUROPEAN SEARCH REPORT

which under Rule 45 of the European Patent Convention shall be considered, for the purposes of subsequent proceedings, as the European search report

Application number: EP 89 11 6857

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | DIALOG. no. 6236313, embase no. 86231376; S.V.S. GOLLAPUDI et al.: "Aryl-fluoroquinolone derivatives A-56619 (difloxacin) and A-56620 inhibit mitogen-induced human mononuclear cell proliferation", & ANTIMICROB. AGENTS CHEMOTHER. (USA), 1986, 30/3 (390-394)<br>* Abstract *<br>------ | 1 | A 61 K 37/02// (A 61 K 37/02 A 61 K 31:47) |

TECHNICAL FIELDS SEARCHED (Int. Cl.4)

A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-13 but as a mixture of compounds, not as method

Claims searched incompletely:

Claims not searched:

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 25-01-1990 | PEETERS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document